(19) Europäisches Patentamt
European Patent Office
Office européen des brevets

(11) EP 4 261 260 A1

(12) EUROPEAN PATENT APPLICATION
published in accordance with Art. 153(4) EPC

(43) Date of publication:
**18.10.2023 Bulletin 2023/42**

(21) Application number: **21902978.2**

(22) Date of filing: **04.10.2021**

(51) International Patent Classification (IPC):
***C09J 11/06*** (2006.01) ***C08L 5/16*** (2006.01)
***C08L 101/02*** (2006.01) ***C09J 129/14*** (2006.01)
***A61L 15/24*** (2006.01) ***A61L 15/26*** (2006.01)
***A61L 15/28*** (2006.01) ***A61L 15/58*** (2006.01)
***A61L 24/00*** (2006.01) ***A61L 24/04*** (2006.01)
***A61L 24/06*** (2006.01) ***A61L 24/08*** (2006.01)
***C09J 7/38*** (2018.01)

(52) Cooperative Patent Classification (CPC):
**A61L 15/24; A61L 15/26; A61L 15/28; A61L 15/58; A61L 24/00; A61L 24/04; A61L 24/06; A61L 24/08; C08L 5/16; C08L 101/02; C09J 7/38; C09J 11/06; C09J 129/14**

(86) International application number:
**PCT/JP2021/036620**

(87) International publication number:
**WO 2022/123874 (16.06.2022 Gazette 2022/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.12.2020 JP 2020203085**

(71) Applicant: **National Institute for Materials Science**
**Tsukuba-shi, Ibaraki 305-0047 (JP)**

(72) Inventor: **TAGUCHI Tetsushi**
**Tsukuba-shi, Ibaraki 305-0047 (JP)**

(74) Representative: **Dehns**
**St. Bride's House**
**10 Salisbury Square**
**London EC4Y 8JD (GB)**

# (54) ADHESIVE SHEET AND METHOD FOR PRODUCING SAME

(57) An adhesive sheet includes cyclodextrin and a derivative polymer in which an alkyl group having a carbon number of 6 to 18 is introduced into a hydrophilic polymer, and at least a part of the alkyl group is in the form of inclusion with the cyclodextrin.

## Description

Technical Field

The present invention relates to an adhesive sheet and a method for producing the same.

Background Art

As an adhesive sheet to be applied to skin, the adhesive sheet including a base material, and a rubber-based adhesive agent, an acrylic adhesive agent, a siliconebased adhesive agent, or the like disposed on the base material has been used. Patent Literature 1 describes an "acrylic adhesive tape having an adhesive agent layer on one side of a support, wherein the adhesive agent layer contains the following (A) to (C) components, the content ratio of the (A) component and the (B) component is 1:0.5 to 1:1.5, and the adhesive agent layer is formed through crosslinking by an external crosslinking agent: (A) a copolymer obtained by copolymerizing a monomer containing a carboxyl group or a hydroxyl group and a (meth)acrylic acid ester as an essential component; (B) a copolymer obtained by copolymerizing a monomer having a nitrogen atom free from a salt structure in a side chain and a (meth)acrylic acid ester as an essential component; and (C) an organic liquid component compatible with the (A) component and the (B) component".

Citation List

Patent Literature

Patent Literature 1: JP 2000-044904 A

Summary of Invention

Technical Problem

However, the present inventor has found that a conventional adhesive agent has insufficient adhesive force against skin or insufficient durability in a moist environment. The present invention addresses a problem of providing an adhesive sheet having excellent adhesive strength against skin and excellent durability in a moist environment, as well as a method for producing the same.

Solution to Problem

As a result of intensive studies to solve the above problem, the present inventor has found that the above problem can be solved by the following configuration.

[1] An adhesive sheet including: cyclodextrin; and a derivative polymer in which an alkyl group having a carbon number of 6 to 18 is introduced into a hydrophilic polymer, wherein at least a part of the alkyl group is in the form of inclusion with the cyclodextrin.

[2] The adhesive sheet according to [1], wherein the alkyl group has a carbon number of 7 to 9.

[3] The adhesive sheet according to [1] or [2], wherein the cyclodextrin is $\alpha$-cyclodextrin or a derivative thereof.

[4] The adhesive sheet according to any one of [1] to [3], wherein the hydrophilic polymer is at least one kind selected from a group consisting of poly(meth)acrylic acid ester, poly(meth)acrylamide, polyvinyl pyrrolidone, polyvinyl alcohol, polyvinyl acetate, polyalkylene glycol, and polyethyleneimine.

[5] The adhesive sheet according to any one of [1] to [4], wherein the derivative polymer is polyvinyl acetal.

[6] The adhesive sheet according to [5], wherein the polyvinyl acetal has a repeating unit represented by Formula 1 mentioned later.

[7] The adhesive sheet according to [6], wherein the $R^1$ alkyl group has a carbon number of 7 to 9.

[8] The adhesive sheet according to [6] or [7], wherein an introduction amount of the alkyl group in the polyvinyl acetal is 0.1 to 8 mol%.

[9] The adhesive sheet according to [6] or [7], wherein an introduction amount of the alkyl group in the polyvinyl acetal is 1 to 4 mol%.

[10] The adhesive sheet according to any one of [1] to [9], wherein a content mass ratio of a content of the cyclodextrin to a content of the derivative polymer is 0.1 to 1.5.

[11] The adhesive sheet according to [10], wherein the content mass ratio is 0.1 to 0.75.

[12] A method for producing the adhesive sheet according to any one of [1] to [11], the method including: preparing a dispersing liquid containing a derivative polymer in which an alkyl group having a carbon number of 6 to 18 is introduced into a hydrophilic polymer, and a solvent; adding cyclodextrin to the dispersing liquid so as to cause at least a part of the alkyl group to be included with the cyclodextrin; and removing the solvent to obtain an adhesive sheet.

[13] The method for producing the adhesive sheet according to [12], further including: introducing the alkyl group into the hydrophilic polymer to obtain the derivative polymer.

[14] The method for producing the adhesive sheet according to [13], wherein the hydrophilic polymer is polyvinyl alcohol, and the introduction is a reaction between the polyvinyl alcohol and aldehyde having the alkyl group.

[15] The adhesive sheet according to any one of [1] to [11], the adhesive sheet for fixing a wearable device.

Advantageous Effects of Invention

According to the present invention, it is possible to provide an adhesive sheet having excellent adhesive strength against skin and excellent durability in a moist environment.

Description of Embodiments

Hereinafter, the present invention will be described in detail.

Components described below may be explained based on representative embodiments of the present invention, but the present invention is not limited to such embodiments.

In the present specification, a numerical range represented by the term "to" means a range including numerical values described before and after the term "to" as a lower limit value and an upper limit value.

In the present specification, the term "inclusion" means a state in which a cyclodextrin molecule has another molecule associated therein in a hydrophobic void (inner cavity) formed thereinside. Therefore, "at least a part of the alkyl group is in the form of inclusion with the cyclodextrin" means a state in which at least a part of the alkyl group is associated with an inner cavity of cyclodextrin.

[Adhesive sheet]

An adhesive sheet according to an embodiment of the present invention includes cyclodextrin and a derivative polymer. This derivative polymer is a polymer in which an alkyl group having a carbon number of 6 to 18 (hereinafter, also referred to as a "specific alkyl group") is introduced into a hydrophilic polymer. At least a part of the alkyl group is included with cyclodextrin.

The reason why the problem in the present invention is solved by the adhesive sheet is not necessarily obvious, but the present inventor makes a conjecture as follows.

The derivative polymer that is a component of the present adhesive sheet constitutes a frame of the adhesive sheet. It is presumed that the specific alkyl group introduced into the hydrophilic polymer has hydrophobicity, and the hydrophobic interaction is likely to be more strongly excited in a moist-affinitive environment, whereby the specific alkyl group has characteristics of easily aggregating in the derivative polymer.

The aggregation of the specific alkyl group by the hydrophobic interaction represents a cause of producing physical entanglement (physical crosslinking) of molecular chains of the derivative polymer.

When physical crosslinking is formed by the specific alkyl group, the frame of the adhesive sheet is strengthened, and the mechanical characteristics and water resistance of the adhesive sheet per se are improved.

In addition, due to the specific alkyl group, the present adhesive sheet exerts an anchor effect to an adherend, particularly a biological tissue having a lipid membrane, and is likely to adhere thereto more strongly.

One of the characteristics of the present adhesive sheet is that at least a part of the specific alkyl group having the above two functions (formation of physical crosslinking/formation of an anchor to an adherend) is included with cyclodextrin.

In a state in which the specific alkyl group is not included, physical crosslinking is likely to occur in the derivative polymer due to hydrophobic interaction. In other words, the derivative polymer not included the specific alkyl group is easy to cure. On the other hand, when physical crosslinking due to the specific alkyl group occurs, the anchor effect is hardly exhibited thereafter, and adhesive strength against the adherend is hardly generated.

In the present adhesive sheet, since the specific alkyl group is included, the formation of physical crosslinking is suppressed. When water comes into contact therewith, cyclodextrin separates from the specific alkyl group. At this time, when the adhesive sheet is in contact with the adherend, the formation of physical crosslinking due to the specific alkyl group and the formation of an anchor to the adherend occur competitively.

Then, in the vicinity of the interface between the adherend and the adhesive sheet, at least a part of the specific

alkyl group is used for adhesion to the adherend, and on the inner side of the adhesive sheet, the specific alkyl group is used for physical crosslinking. That is, the present adhesive sheet firmly adheres to an adherend by an anchor effect due to the specific alkyl group, and the sheet per se is also cured by physical crosslinking due to the specific alkyl group, whereby the water resistance is also enhanced.

As described above, the present adhesive sheet has excellent adhesiveness to an adherend in a moist environment (in the presence of moisture), and furthermore, the adhesive sheet has a property of increasing the water resistance thereof since physical crosslinking is formed therein.

In general, an adhesive sheet using a water-soluble adhesive agent has a problem that, if the adhesive sheet adheres to an adherend in a moist environment and is dried and cured once, and then, the adhesive sheet is brought into contact with moisture again, the adhesive agent is softened to decrease the adhesive strength, and the adhesive sheet peels off in some cases.

On the other hand, in a case of the present adhesive sheet, once the adhesive sheet adheres, even if the adhesive sheet is brought into contact with moisture again, the adhesive strength does not decrease, but rather, physical crosslinking further progresses, and the water resistance of the sheet further increases.

In addition, a general adhesive sheet is often composed of a base material and an adhesive agent layer formed on the base material. When the adhesive agent layer has tack or is unstable, if no base material is provided on one side thereof, the handleability is significantly deteriorated, or mechanical strength tends to be insufficient only with the adhesive layer, whereby it is difficult to use the adhesive agent layer alone as an "adhesive sheet".

On the other hand, the present adhesive sheet has almost no tack at the beginning, and is easily handled even without a base material provided therein. When the present adhesive sheet comes into contact with an adherend in a wet condition, an interface with the adherend functions like an adhesive agent layer, and an inner side therefrom functions like a base material by the formation of physical crosslinking.

In addition, in a general adhesive sheet, when the adhesive agent layer has tack, in a case of winding the adhesive sheet to form a roll, it is necessary to attach a release sheet (separator) to an adhesive surface in advance or to perform a treatment of preventing adhesion on the surface opposed to the base material in order to avoid unintended adhesion by the adhesive agent layer.

On the other hand, since the present adhesive sheet comes into contact with an adherend in a wet condition to generate an adhesive strength, even in a case of winding the adhesive sheet to form a roll, the above-mentioned treatment is not required.

Furthermore, in the present adhesive sheet, a surface in contact with an adherend functions like an adhesive agent layer, and thus, for example, if both surfaces of the adhesive sheet are brought into contact with an adherend in a wet condition, the adhesive sheet can also be used as a double-sided adhesive sheet.

In a general adhesive sheet, for double-side adhesion, it is necessary to provide adhesive agent layers on both surfaces of a base material in advance, and the configuration and the manufacturing method thereof are different from those of an adhesive sheet for single-side adhesion.

The present adhesive sheet is different from a conventional adhesive sheet in that it is usable even without a base material, and thus can be used for single-side adhesion or both-side adhesion in accordance with the application, without any special treatment or preparation. Hereinafter, components of the present adhesive sheet will be described in detail.

<Derivative polymer>

The present adhesive sheet includes a derivative polymer obtained by introducing an alkyl group having a carbon number of 6 to 18 into a hydrophilic polymer. Hereinafter, an alkyl group having a carbon number of 6 to 18 may be referred to as a "specific alkyl group".

The content of the derivative polymer in the present adhesive sheet is not particularly limited, but is preferably 20 mass% or more, more preferably 40 mass% or more, still more preferably 55 mass% or more, and is preferably 90 mass% or less, more preferably 80 mass% or less, still more preferably 70 mass% or less when the total mass of the adhesive sheet is defined to be 100 mass%.

The present adhesive sheet may contain one kind of derivative polymer alone, or may contain two or more kinds thereof. When the present adhesive sheet contains two or more kinds of derivative polymers, the total content thereof preferably falls within the above-mentioned numerical range.

The hydrophilic polymer is not particularly limited, but examples thereof include alginate, polyethylene oxide, polyethylene glycol, polyvinyl acetate, polyvinyl pyrrolidone, methyl cellulose, ethyl cellulose, hydroxypropyl cellulose, hydroxyethyl cellulose, hydroxyethyl methyl cellulose, hydroxypropyl methyl cellulose, polyacrylic acid, carboxymethyl cellulose sodium, polyvinyl alcohol, polyethylene oxide, ethylene oxide-propylene oxide copolymer, collagen, gelatin, albumin, polyamine, polyphosphazene, polysaccharide, chitin, chitosan, poly(meth)acrylic acid ester, poly(meth)acrylamide, ethylene vinyl alcohol (ethylene-vinyl alcohol copolymer), polyalkylene glycol, polyethyleneimine, glycosaminoglycan (For example, hyaluronic acid, chondroitin sulfate, and the like), proteoglycan, xanthan gum, carrageenan, gellan gum, guar gum, locust bean gum, sacran, and the like.

Among them, from the viewpoint of obtaining an adhesive sheet having more advantageous effects of the present invention, the hydrophilic polymer is preferably at least one kind selected from a group consisting of polyvinyl alcohol, polyvinyl acetate, polyvinyl pyrrolidone, poly(meth)acrylic acid ester, poly(meth)acrylamide, polyalkylene glycol, polyethyleneimine, and polyamine, and more preferably at least one kind selected from a group consisting of polyvinyl alcohol and polyvinyl acetate.

A method for obtaining a derivative polymer by introducing a specific alkyl group into a hydrophilic polymer is not particularly limited, and a known method can be used. Examples of such a method include a method for causing a compound having a site capable of reacting with a reactive substituent (for example, a hydroxy group, a carboxy group, an amino group, and the like.) of the hydrophilic polymer, and a specific alkyl group, to react with the hydrophilic polymer. Examples of such a compound include an alcohol, an amine, an aldehyde, a ketone, and the like having a specific alkyl group.

The specific alkyl group is an alkyl group having a carbon number of 6 to 18. When the carbon number in the alkyl group is less than 6, the anchor effect is not sufficiently exhibited, and it is impossible to obtain sufficient adhesive strength against an adherend. In addition, physical crosslinking becomes insufficient, and it is impossible to obtain the water resistance of the adhesive sheet.

On the other hand, when the carbon number exceeds 18, the hydrophobicity becomes too high and the cohesive force becomes too strong, and as a result, it is impossible to obtain sufficient adhesiveness.

From the viewpoint of obtaining an adhesive sheet having more advantageous effects of the present invention, the carbon number in the specific alkyl group is preferably 7 or more, more preferably 8 or more, and preferably 14 or less, more preferably 12 or less, still more preferably 10 or less, particularly preferably 9 or less.

The specific alkyl group may be straight-chain or branched-chain, but from the viewpoint of obtaining more advantageous effects of the present invention, the specific alkyl group is preferably straight-chain.

The introduction amount of the alkyl group in the derivative polymer is not particularly limited, but is preferably 0.1 mol% or more, more preferably 1 mol% or more, and preferably 8 mol% or less, more preferably 4 mol% or less.

When the introduction amount of the alkyl group is 1 to 4 mol%, the resulting adhesive sheet has further excellent peel strength.

In the present specification, the introduction amount of the alkyl group means a value (mol%) obtained from $^1$H-NMR measurement result of the derivative polymer.

For example, when the derivative polymer is polyvinyl acetal, the introduction amount of the alkyl group is calculated using the following method.

First, a 0.5% (w/v) solution of polyvinyl acetal and dimethyl sulfoxide-$d_6$ is prepared. The measurement is performed thereon by use of a $^1$H-NMR apparatus (trade name "JNM-ECS-400", manufactured by JEOL Ltd.). The measurement is performed at 25 °C. Each sample is scanned eight times. From the obtained result, the introduction amount of the alkyl group is calculated using the following calculation formula.

$$\text{(Formula) Introduction amount (mol\%) of alkyl group} = \{(\text{peak area of -CH}_3\text{ protons})/3\}/(\text{peak area of -CH- protons}) \times 100$$

Here, the peak area of -CH- protons is the product of peaks derived from $\alpha$CH protons of a skeleton at 3.08 ppm.

The peak area of the -$CH_3$ protons is a peak area at 0.75 ppm derived from $CH_3$ of the alkyl group.

(Preferred form of derivative polymer)

From the viewpoint of obtaining an adhesive sheet having more advantageous effects of the present invention, a preferred form of the derivative polymer is preferably polyvinyl acetal (hereinafter, also referred to as a "derivative polymer 1") having a repeating unit (hereinafter, also referred to as "unit 1") represented by the following Formula 1.

[Chemical formula 1]

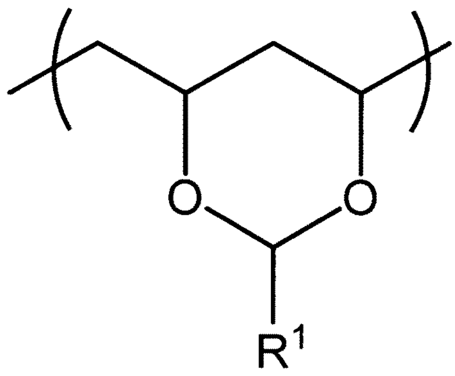

(1)

In the above formula, $R^1$ represents an alkyl group having a carbon number of 6 to 18 (specific alkyl group).

The carbon number in the $R^1$ alkyl group is preferably 7 or more, more preferably 8 or more, and preferably 14 or less, more preferably 12 or less, still more preferably 10 or less, particularly preferably 9 or less. The $R^1$ alkyl group may be straight-chain or branched-chain, but from the viewpoint of obtaining more advantageous effects of the present invention, the $R^1$ alkyl group is preferably straight-chain.

The derivative polymer 1 may further have a repeating unit other than that represented by Formula 1. Examples of such a repeating unit include repeating units represented by the following Formula 2 and Formula 3 (hereinafter, also referred to as "unit 2" and "unit 3", respectively).

[Chemical formula 2]

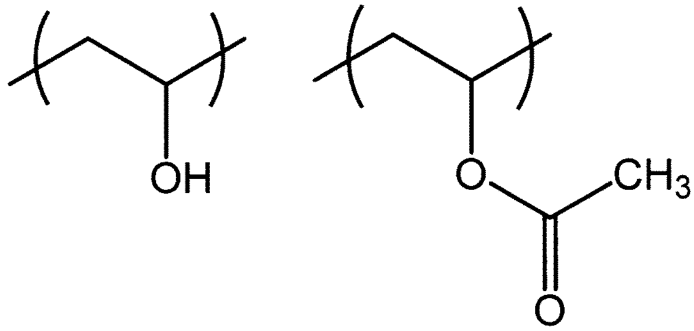

(2) (3)

The content of respective units in the derivative polymer 1 is not particularly limited, but the unit 1 is preferably 0.1% or more, more preferably 1% or more, and is preferably 20% or less, more preferably 15% or less, further preferably 8% or less, particularly preferably 4.5% or less when the number of total respective units is defined to be 100%.

The content of the unit 2 in the derivative polymer 1 is not particularly limited, but, if the derivative polymer 1 contains the unit 2, is preferably 72% or more, more preferably 76.5% or more, further preferably 83% or more, particularly preferably 86% or more, and preferably 98.9% or less, and more preferably 98% or less when the number of total respective units is defined to be 100%.

The content of the unit 3 in the derivative polymer 1 is not particularly limited, but, if the derivative polymer 1 contains the unit 3, is preferably 0.1% or more, more preferably 1% or more, and preferably 9.5% or less, more preferably 9% or less, still more preferably 8.5% or less, particularly preferably 8% or less when the number of total respective units is defined to be 100%.

The molecular weight of the derivative polymer 1 is not particularly limited, but is generally preferably 10,000 to 200,000, and more preferably 50,000 to 100,000.

The method for producing the derivative polymer 1 is not particularly limited, and a known production method can be applied. Typically, the derivative polymer 1 can be obtained by condensing polyvinyl alcohol obtained by saponifying polyvinyl acetate with an aldehyde in the presence of an acid catalyst. In this case, the specific alkyl group in the resulting derivative polymer 1 is derived from the aldehyde. Therefore, the derivative polymer 1 having a desired alkyl group can be produced by appropriately changing the type and the content ratio of aldehyde to be used.

<Cyclodextrin>

The adhesive sheet contains cyclodextrin. Cyclodextrin is a cyclic compound in which D-glucose units are circularly bonded through α-1,4-glucoside bond, and can be produced by acting an enzyme such as cyclodextrin glucanotransferase on starch and/or a hydrolyzate of starch.

As cyclodextrin, cyclodextrin having six (α-type), seven (β-type), or eight (γ-type) constituent glucoses, for example, can be used, and derivatives thereof can also be used.

Among them, α-cyclodextrin or a derivative thereof is preferable in that the size of the inner cavity thereof is more suitable for including the specific alkyl group.

Examples of derivatives of α-cyclodextrin include methyl α-cyclodextrin, butyl α-cyclodextrin, 2-hydroxypropyl α-cyclodextrin, acetyl α-cyclodextrin, succinyl α-cyclodextrin, glucosyl α-cyclodextrin, maltosyl α-cyclodextrin, α-cyclodextrin carboxymethyl ether, phosphate α-cyclodextrin, carboxymethyl α-cyclodextrin, and the like.

Examples of derivatives of β-cyclodextrin include methyl-β-cyclodextrin (MBCD), (2-hydroxypropyl)-β-cyclodextrin (HPBCD), carboxymethyl-β-cyclodextrin, carboxymethyl-ethyl-β-cyclodextrin, diethyl-β-cyclodextrin, dimethyl-β-cyclodextrin, glucosyl-β-cyclodextrin, hydroxybutenyl-β-cyclodextrin, hydroxyethyl-β-cyclodextrin, maltosyl-β-cyclodextrin, random methyl-β-cyclodextrin, sulfobutyl ether-β-cyclodextrin, 2-selenium cross-linked β-cyclodextrin, 2-tellurium cross-linked β-cyclodextrin, and the like.

Examples of derivatives of γ-cyclodextrin include 2-hydroxyethyl-γ-cyclodextrin, 2-hydroxypropyl-γ-cyclodextrin, butyl-γ-cyclodextrin, 3A-amino-3A-deoxy- (2AS, 3AS)-γ-cyclodextrin, mono-2-O-(p-toluenesulfonyl)-γ-cyclodextrin, mono-6-O-(p-toluenesulfonyl)-γ-cyclodextrin, mono-6-O-mesitylenesulfonyl-γ-cyclodextrin, octakis (2,3,6-tri-O-methyl)-γ-cyclodextrin, octakis (2,6-di-O-phenyl)-γ-cyclodextrin, octakis (6-O-t-butyldimethylsilyl)-γ-cyclodextrin, octakis (2,3,6-tri-O-acetyl)-γ-cyclodextrin, and the like.

The content of cyclodextrin in the present adhesive sheet is not particularly limited, but, from the viewpoint of obtaining an adhesive sheet having more advantageous effects of the present invention, is preferably 10 mass% or more, more preferably 20 mass% or more, still more preferably 30 mass% or more, and is preferably 80 mass% or less, more preferably 60 mass% or less, still more preferably 50 mass% or less, particularly preferably 45 mass% or less when the total mass of the adhesive sheet is defined to be 100 mass%.

In addition, the content mass ratio of the content of cyclodextrin to the content of the derivative polymer in the adhesive sheet (the content of cyclodextrin/ the content of derivative polymer) is preferably 0.01 or more, more preferably 0.1 or more, further preferably 0.3 or more, and preferably 3.0 or less, more preferably 1.5 or less, further preferably 1.0 or less, particularly preferably 0.9 or less, and most preferably 0.75 or less.

In particular, when the above-mentioned content mass ratio is 0.1 to 0.75, the resulting adhesive sheet has further excellent peel strength and further excellent stability in water.

Note that the adhesive sheet may contain one kind of cyclodextrin alone, or may contain two or more kinds thereof. When a composite contains two or more kinds of cyclodextrin, the total content thereof preferably falls within the above-mentioned numerical range.

The adhesive sheet may contain other components within the range in which the effect of the present invention is provided.

[Adhesive sheet production method]

A method for producing an adhesive sheet is not particularly limited, but preferably includes the following steps so that the adhesive sheet can be more easily produced: preparing a dispersing liquid containing a derivative polymer in which an alkyl group having a carbon number of 6 to 18 is introduced into a hydrophilic polymer, and a solvent;

adding cyclodextrin to the dispersing liquid so as to cause at least a part of the alkyl group to be included with the cyclodextrin; and
removing the solvent to obtain an adhesive sheet.

The solvent used for producing the dispersing liquid preferably contains water. The solvent preferably contains a water-soluble organic solvent in addition to water. The water-soluble organic solvent is not particularly limited, but examples thereof include: $C_1$ to $C_6$ alcohols such as methanol, ethanol, isopropyl alcohol, and ethylene glycol; and aprotic polar solvents such as dimethylformamide, acetone, tetrahydrofuran, dimethylsulfoxide, and N-methylpyrrolidone.

Among them, from the viewpoint of easy handling and lower toxicity, $C_1$ to $C_6$ alcohols are preferable as the organic solvent, and especially, ethanol is preferable.

When the solvent contains $C_1$ to $C_6$ alcohols, the content thereof is not particularly limited, but is preferably 10 to 90% and more preferably 15 to 50% on a volume basis.

As a method for preparing a dispersing liquid, from the viewpoint of convenience, it is preferable that a derivative

polymer is dispersed in a solvent in advance to obtain a dispersing liquid, and cyclodextrin is added to the dispersing liquid.

Thereafter, a method for including the alkyl group in the derivative polymer with cyclodextrin is not particularly limited, and a known method can be used. Among them, a heating method is preferable.

Examples of the inclusion method include a method for heating the dispersing liquid at 60 to 120 °C for 1 to 60 minutes. At this time, it is preferable to pressurize the dispersing liquid.

A method for obtaining an adhesive sheet from the dispersing liquid after the inclusion of the alkyl group is not particularly limited. For example, a dispersing liquid layer may be formed on a support or a temporary support using the dispersing liquid, and then, the solvent may be removed from the dispersing liquid layer.

A method for forming the dispersing liquid layer is not particularly limited, and it is possible to use a known coating or printing technique, and the like. In addition, a method for injecting the dispersing liquid into a mold which may or may not have a curved surface can also be used.

For example, in one example of the method for obtaining the adhesive sheet, in a case of using a roll coater, a dispersing liquid layer is formed on a temporary support by use of a gravure coater, a reverse coater, a slot die coater, a knife coater, a comma coater, a bar coater or the like, and dried in an oven, and subsequently, the temporary support is peeled off.

As a method for removing the solvent from the obtained dispersing liquid layer, a known method can be used. Examples thereof include a method in which a dispersing liquid layer is retained at 10 to 100 °C for 1 minute to 48 hours to dry the solvent. At this time, the pressure may be reduced.

The present adhesive sheet reacts with moisture on the surface of the adherend to generate adhesive strength. Therefore, the obtained adhesive sheet may be wound around a winding core to form a roll without a release paper or the like being attached thereto.

The thickness of the adhesive sheet is not particularly limited, but is generally preferably 5 to 200 μm.

[Application of adhesive sheet]

The present adhesive sheet has excellent adhesive strength against a living tissue and excellent water resistance, and therefore can be used for fixing a wearable device.

In addition, as shown in Examples, since the present adhesive sheet has excellent ion permeability and hardly allows proteins to permeate therethrough, the present adhesive sheet exhibits excellent performance even when a wearable device is for measuring biological information.

A method for fixing a wearable device using the adhesive sheet is not particularly limited, and examples thereof include the following fixation methods.

First, a wearable device such as a biological information monitor is placed on the skin. Here, the present adhesive sheet larger than the contour of the wearable device in a plan view is used and caused to adhere to the skin so as to cover the wearable device. The present adhesive sheet has an excellent water vapor transmission rate, excellent ion permeability, and the like, and thus, even when the biological information monitor is fixed through this method, the function thereof is hardly impaired, and stuffiness is prevented.

In addition, in the present adhesive sheet, since physical crosslinking progresses in a moist environment to form a strong film, it is also possible to form a device directly on the adhesive sheet by applying a printed electronics technology or the like. The wearable device can be fixed by being attached the adhesive sheet to the skin in a state where the wearable device is formed.

In addition, in a case where the housing of the wearable device has a hydrophilic surface (for example, formed of the hydrophilic polymer described above), the present adhesive sheet is disposed between the skin and the wearable device, and both surfaces are caused to adhere to each other, whereby it is possible to cause the skin and the wearable device to adhere to each other via the present adhesive sheet.

Since the present adhesive sheet has an excellent water vapor transmission rate, excellent ion permeability, and the like, and thus, even when a device such as a biological information monitor is fixed through this method, the function of the device can be sufficiently exhibited.

Examples

Hereinafter, the present invention will be described in more detail based on Examples. Materials, usage amounts, ratios, processing detail, processing procedures, and the like shown in the following examples can be appropriately changed without departing from the gist of the present invention. Therefore, the scope of the present invention should not be restrictively interpreted by the following examples.

(Synthesis of C9-PVA)

Water and DMSO (dimethyl sulfoxide) were mixed at a volume ratio of 1:3, and hydrochloric acid was further added thereto to prepare a solvent having a final concentration of 0.01 M. Polyvinyl alcohol (Mw = 88,000, saponification ratio > 98.5%, manufactured by Nacalai tesque, Inc.) was added to the solvent so as to achieve 5% w/v and mixed therewith to obtain a mixed solution.

Next, nonanal ($C_9$, however, the alkyl group introduced as $R^1$ in Formula 1 is $C_8$) prepared in such an amount that the introduction amounts of the alkyl group were 2.5 mol% and 5 mol% was added to the mixed solution, and the resultant solution was stirred for one hour while the liquid temperature was maintained at 50 °C using a reflux condenser. The amounts of the introduced alkyl group were confirmed by $^1$H-NMR. As a result, the introduction amounts of the alkyl group were 2.5 mol% and 5 mol%, respectively, and the consumption amounts of the hydroxy group were 5 mol% and 10 mol%, respectively.

Hereinafter, a product obtained through this reaction is referred to as "C9-PVA". C9-PVA has unit 1, and $R_1$ in Formula 1 is a straight-chain alkyl group (octyl group) having a carbon number of 8.

After the reaction, the resulting C9-PVA solution was added to 600 mL of cold ethanol while being stirred for one hour. Unreacted aldehyde and DMSO in the solvent were removed through this ethanol cleansing. Finally, the precipitated C9-PVA was evaporated as white crystals under vacuum. Next, the obtained crystals were finely pulverized using a crusher (trade name "Wonder Crusher WC-3", manufactured by Osaka Chemical Co., Ltd.).

(Preparation of adhesive sheet)

First, α-cyclodextrin (hereinafter, referred to as "α-CD", manufactured by FUJIFILM Wako Pure Chemical Corporation) was added to a 1% w/v "C9-PVA" dispersing liquid (40% v/v ethanol aqueous solution), and heated in an autoclave (trade name "LSX-500", manufactured by TOMY) at 90 °C for 10 minutes.

At this time, the addition amount of α-CD was adjusted so that the content mass ratio of the α-CD content to the C9-PVA content (α-CD/C9-PVA) in the adhesive sheet was 0.5 or 1.

Note that the inclusion of α-CD with the alkyl group was confirmed based on the fact that, when nonanal and α-CD were added into 40% ethanol and processed under the same conditions as described above, a white precipitate was generated (inclusion complex).

The resulting solution was then stirred at 25 °C overnight. The solution was then poured into a silicone mold having a thickness of 1 mm and dried overnight at 25 °C in a clean bench (manufactured by AS ONE Corporation).

After drying, the adhesive sheet was peeled off from the silicone mold, and the thickness was measured by use of a micrometer ("MDC-MX", Mitutoyo Corporation).

(Adhesiveness: T-peel test)

AT-peel test was performed using the skin of a pig as an adherend. First, the skin of the pig used was purchased from Tokyo Shibaura Organ Co., Ltd. Prior to the experiment, the skin of the pig was washed three times with PBS (phosphate buffered saline) and the surface thereof was shaved with a clipper (trade name "Thrive model 515 R", manufactured by Daito Electric Industrial Co., Ltd.). The skin of the pig was used within 48 hours after slaughter.

The measurement in the T-peel test of the adhesive sheet was performed in accordance with ASTM F 2256-05.

First, the prepared skin of the pig was cut into a rectangle of 200 mm × 30 mm, and excess moisture was removed using a mesh. Thereafter, 80 μL of a 40% v/v ethanol aqueous solution was sprayed onto the skin of the pig, and an adhesive sheet (100 mm × 15 mm, thickness: 15 μm) was attached thereto.

After attachment, drying was performed at 25 °C for 15 minutes. The skin of the pig was fixed to a stage, and the adhesive sheet was peeled off (200 mm/min) while one end of the adhesive sheet is being held with a jig so that the angle formed between the direction of peel-off (peeled adhesive sheet) and the surface of the skin of the pig was 90 degrees, and the peel strength was measured.

Tests were performed on each sample five times, and the arithmetic average thereof was calculated to obtain the average peel strength (N/m). The obtained average peel strength was evaluated according to the following criteria. The results are shown in a column of "peel strength" in Table 1.

Note that, as a reference, the same test was also performed with the "3M Scotch Tape (trade name)" and the "Tegaderm Film (trade name)".

· Evaluation criteria

AA: Average peel strength was 8.00 N/m or more.
A: Average peel strength was 6.00 N/m or more and less than 8.00 N/m.
B: Average peel strength was 4.00 N/m or more and less than 6.00 N/m.
C: Average peel strength was 3.00 N/m or more and less than 4.00 N/m.
D: Average peel strength was less than 3.00 N/m.

(Stability in water)

40% (v/v) ethanol was sprayed onto the pig skin having a size of 60 mm × 60 mm to wet the skin, and an adhesive sheet having a size of about 30 mm × 30 mm and a thickness of 15 μm was attached thereto to prepare a laminate. Next, this laminate was immersed in a container containing 300 mL of physiological saline (liquid temperature of 37 °C) and retained for 24 hours while being stirred at a rotation speed of 200 rpm, and then, the time until peel-off was measured. Three different test pieces were prepared for each of the adhesive sheets, and average values thereof were used as the results. The results were evaluated according to the following criteria. The results are shown in a column of "stability in water" in Table 1.

· Evaluation criteria

AA: Stability in water was 125 seconds or more.
A: Stability in water was 75 seconds or more and less than 125 seconds.
B: Stability in water was 25 seconds or more and less than 75 seconds.
C: Stability in water was less than 25 seconds.

Note that no peel-off or the like was observed in the adhesive sheet of Example 1 even after a lapse of 24 hours since the start of the test.

(Water vapor transmission rate)

The water vapor transmission rate of the adhesive sheet was measured in accordance with the ASTM 96 procedure B. First, a screw cap vial having a bottom area of 3.14 $cm^2$ was prepared, and distilled water was weighed and added thereto in predetermined amounts. Next, an opening of the vial was closed with the adhesive sheet, and the adhesive sheet was fixed in that state.
In this state, the temperature was retained at 37 °C, and the amount of water evaporated from the vial was quantified.
The water vapor transmission rate (WVTR) was calculated using the following formula.

$$\text{(Formula) WVTR} = V/T$$

Note that V represents volume change (decrease) of distilled water, and T represents incubation time.
Note that tests were performed on each adhesive sheet three times, and the arithmetic mean thereof was used as a result. The results were evaluated according to the following criteria. The results are shown in a column of "stability in water" in Table 1.

· Evaluation criteria

A: The water vapor transmission rate was 0.50 mL/day or more.
B: The water vapor transmission rate was 0.10 mL/day or more, and less than 0.50 mL/day.
C: The water vapor transmission rate was less than 0.10 mL/day.

(Ion permeability)

A 6-well plate with a transwell (registered trademark) insert having a diameter of 24 mm was prepared. The transwell insert has a hole having a diameter of 8 μm at the bottom, and each adhesive film was formed as a cast film

so as to close the hole. Next, an aqueous NaCl solution having a concentration of 100 mg/mL was added into the insert, and 3 mL of deionized water was added to each well of the 6-well plate, and then, the plate was incubated at 37 °C.

NaCl leaching into deionized water in the well after a lapse of one hour since the start of the test was quantified by ICP-OES (Inductively coupled plasma optical emission spectrometry). Tests were performed five times, and the arithmetic mean was calculated and used as the result. The results were evaluated according to the following criteria. Table 1 shows the results.

· Evaluation criteria

A: The amount of leached NaCl was 30 mg/mL or more.
B: The amount of leached NaCl was 20 mg/mL or more and less than 30 mg/mL.
C: The amount of leached NaCl was less than 20 mg/mL.

(Protein transmission rate)

A 6-well plate with a transwell (registered trademark) insert having a diameter of 24 mm was prepared. The transwell insert has a hole having a diameter of 8 μm at the bottom, and each adhesive film was formed as a cast film so as to close the hole.

Next, HSA (Human serum albumin) having a concentration of 2 mg/mL was added into the insert, 3 mL of D-PBS (Dulbecco's Phosphate Buffered Saline) was added to each well of the 6-well plate, and then, the plate was incubated at 37 °C.

HSA leaching into the buffering solution in the well after a lapse of one hour since the start of the test was quantified by a micro BCA assay. Tests were performed five times, and the arithmetic mean was calculated and used as the result. The results were evaluated according to the following criteria. Table 1 shows the results.

. Evaluation criteria

A: The amount of leached HSA was less than 5 μg/mL.
B: The amount of leached HSA was 5 μg/mL or more and less than 15 μg/mL.
C: The amount of leached HSA was 15 μg/mL or more.

[Table 1]

| Table 1 | Sample name | Peel strength | Stability in water | Water vapor transmission rate | Protein transmission rate (lh) | Ion permeability (lh) |
|---|---|---|---|---|---|---|
| Comparative Example 1 | PVA | D | B | A | C | C |
| Comparative Example 2 | α-CD/PVA (w/w = 1) | B | C | A | B | A |
| Comparative Example 3 | 2.5C9-PVA | D | A | A | A | B |
| Example 1 | α-CD/2.5C9-PVA (w/w = 0.5) | AA | AA | A | A | A |
| Example 2 | α-CD/2.5C9-PVA (w/w = 1) | A | B | | A | B |
| Example 3 | α-CD/5C9-PVA (w/w = 1) | B | B | | | |
| Reference Example 1 | Scotch™ tape | C | AA | C | | |

(continued)

| Table 1 | Sample name | Peel strength | Stability in water | Water vapor transmission rate | Protein transmission rate (lh) | Ion permeability (lh) |
|---|---|---|---|---|---|---|
| Reference Example 2 | Tegaderm™ film | B | AA | B | | |

In Table 1, the meanings of notations in columns of sample names are as follows.

- "PVA": a sheet made of polyvinyl alcohol
- "α-CD/PVA (w/w = 1)": a sheet prepared in the same manner as in Example 3 except that the PVA was used instead of the derivative polymer
- "2.5C9-PVA": a sheet made of C9-PVA having the introduction amount of the alkyl group being adjusted to 2.5 mol%
- "α-CD/2.5C9-PVA (w/w = 0.5)": an adhesive sheet having the content mass ratio between α-CD and C9-PVA in which the introduction amount of the alkyl group is 2.5 mol%, being adjusted to 1:2
- "α-CD/2.5C9-PVA (w/w = 1)": an adhesive sheet having the content mass ratio between α-CD and C9-PVA in which the introduction amount of the alkyl group is 2.5 mol%, being adjusted to 1:1
- "α-CD/5C9-PVA (w/w = 1)": an adhesive sheet having the content mass ratio between α-CD and C9-PVA in which the introduction amount of the alkyl group is 5 mol%, being adjusted to 1:2

From the results in Table 1, the adhesive sheet of Example 1 exhibited excellent peel strength and excellent stability in water as compared with the adhesive sheet of Comparative Example 1 made of a hydrophilic polymer (PVA) containing no cyclodextrin and having no specific alkyl group introduced thereinto.
Furthermore, the adhesive sheet of Example 1 exhibited excellent stability in water as compared with the adhesive sheet of Comparative Example 2 made of cyclodextrin and a hydrophilic polymer (PVA) having no specific alkyl group introduced thereinto.
Furthermore, the adhesive sheet of Example 1 exhibited excellent peel strength as compared with the adhesive sheet of Comparative Example 3 made of the derivative polymer having the specific alkyl group introduced thereinto and containing no cyclodextrin.
In addition, the adhesive sheet of Example 1 exhibited more excellent peel strength as compared with commercially available products (Scotch Tape and Tegaderm Film).
In addition, the adhesive sheet of Example 1 having a content mass ratio of the content of cyclodextrin to the content of the derivative polymer in the adhesive sheet of 0.1 to 0.75 had more excellent peel strength and more excellent stability in water as compared with the adhesive sheet of Example 2.
In addition, the adhesive sheet of Example 2 in which the introduction amount of the alkyl group was 1 to 4 mol% exhibited more excellent peel strength as compared with the adhesive sheet of Example 3.

Industrial Applicability

Since the present adhesive sheet has both excellent adhesive strength and excellent durability in a moist environment, it is suitable for usage for being attached to an adherend such as skin. In addition, since it has an excellent water vapor transmission rate, an ion transmission rate, and the like, the adhesive sheet is also suitable for fixing a wearable device.

## Claims

1. An adhesive sheet comprising:

    cyclodextrin; and
    a derivative polymer in which an alkyl group having a carbon number of 6 to 18 is introduced into a hydrophilic polymer, wherein
    at least a part of the alkyl group is in the form of inclusion with the cyclodextrin.

2. The adhesive sheet according to claim 1, wherein the alkyl group has a carbon number of 7 to 9.

3. The adhesive sheet according to claim 1 or 2, wherein the cyclodextrin is α-cyclodextrin or a derivative thereof.

4. The adhesive sheet according to any one of claims 1 to 3, wherein the hydrophilic polymer is at least one kind selected from a group consisting of poly(meth)acrylic acid ester, poly(meth)acrylamide, polyvinyl pyrrolidone, polyvinyl alcohol, polyvinyl acetate, polyalkylene glycol, and polyethyleneimine.

5. The adhesive sheet according to any one of claims 1 to 3, wherein the derivative polymer is polyvinyl acetal.

6. The adhesive sheet according to claim 5, wherein the polyvinyl acetal has a repeating unit represented by the following Formula 1.

[Chemical formula 1]

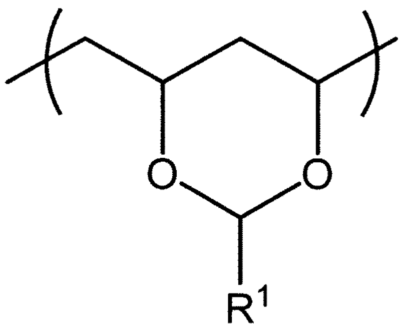

(1)

(In Formula 1, $R^1$ represents an alkyl group having a carbon number of 6 to 18.)

7. The adhesive sheet according to claim 6, wherein the $R^1$ alkyl group has a carbon number of 7 to 9.

8. The adhesive sheet according to claim 6 or 7, wherein an introduction amount of the alkyl group in the polyvinyl acetal is 0.1 to 8 mol%.

9. The adhesive sheet according to claim 6 or 7, wherein an introduction amount of the alkyl group in the polyvinyl acetal is 1 to 4 mol%.

10. The adhesive sheet according to any one of claims 1 to 9, wherein a content mass ratio of a content of the cyclodextrin to a content of the derivative polymer is 0.1 to 1.5.

11. The adhesive sheet according to claim 10, wherein the content mass ratio is 0.1 to 0.75.

12. A method for producing the adhesive sheet according to any one of claims 1 to 11, the method comprising:

preparing a dispersing liquid containing a derivative polymer in which an alkyl group having a carbon number of 6 to 18 is introduced into a hydrophilic polymer, and a solvent;
adding cyclodextrin to the dispersing liquid so as to cause at least a part of the alkyl group to be included with the cyclodextrin; and
removing the solvent to obtain an adhesive sheet.

13. The method for producing the adhesive sheet according to claim 12, the method further comprising: introducing the alkyl group into the hydrophilic polymer to obtain the derivative polymer.

14. The method for producing the adhesive sheet according to claim 13, wherein the hydrophilic polymer is polyvinyl alcohol, and the introduction is a reaction between the polyvinyl alcohol and aldehyde having the alkyl group.

15. The adhesive sheet according to any one of claims 1 to 11, the adhesive sheet for fixing a wearable device.

**INTERNATIONAL SEARCH REPORT**

International application No.
**PCT/JP2021/036620**

**A. CLASSIFICATION OF SUBJECT MATTER**

***C09J 11/06***(2006.01)i; ***C08L 5/16***(2006.01)i; ***C08L 101/02***(2006.01)i; ***C09J 129/14***(2006.01)i; ***A61L 15/24***(2006.01)i; ***A61L 15/26***(2006.01)i; ***A61L 15/28***(2006.01)i; ***A61L 15/58***(2006.01)i; ***A61L 24/00***(2006.01)i; ***A61L 24/04***(2006.01)i; ***A61L 24/06***(2006.01)i; ***A61L 24/08***(2006.01)i; ***C09J 7/38***(2018.01)i

FI: C09J7/38; A61L15/26 100; A61L15/58 100; A61L15/28 100; A61L24/00 300; A61L24/04 100; A61L24/06; A61L24/04 200; A61L24/08; C08L5/16; C08L101/02; A61L15/24 100; C09J11/06; C09J129/14

According to International Patent Classification (IPC) or to both national classification and IPC

**B. FIELDS SEARCHED**

Minimum documentation searched (classification system followed by classification symbols)

C09J11/06; C08L5/16; C08L101/02; C09J129/14; A61L15/24; A61L15/26; A61L15/28; A61L15/58; A61L24/00; A61L24/04; A61L24/06; A61L24/08; C09J7/38

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | WO 2020/013215 A1 (UNIV TOKYO) 16 January 2020 (2020-01-16) claims, examples 1-23 | 1-15 |
| A | JP 2020-105360 A (LINTEC CORP) 09 July 2020 (2020-07-09) claims, examples 1-7 | 1-15 |
| A | JP 2019-189819 A (LINTEC CORP) 31 October 2019 (2019-10-31) claims, examples 1-7 | 1-15 |
| A | JP 2015-192109 A (FUJIFILM CORP) 02 November 2015 (2015-11-02) claims, examples 1-16 | 1-15 |
| A | JP 2010-138259 A (LINTEC CORP) 24 June 2010 (2010-06-24) claims, examples 1-7 | 1-15 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 November 2021** | **07 December 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No. **PCT/JP2021/036620**

**C. DOCUMENTS CONSIDERED TO BE RELEVANT**

| Category* | Citation of document, with indication, where appropriate, of the relevant passages | Relevant to claim No. |
|---|---|---|
| A | JP 2009-292727 A (ADVANCED SOFTMATERIALS INC) 17 December 2009 (2009-12-17) claims, example 1-3 | 1-15 |

Form PCT/ISA/210 (second sheet) (January 2015)

**INTERNATIONAL SEARCH REPORT**
**Information on patent family members**

International application No.
**PCT/JP2021/036620**

| Patent document cited in search report | Publication date (day/month/year) | Patent family member(s) | Publication date (day/month/year) |
|---|---|---|---|
| WO 2020/013215 A1 | 16 January 2020 | EP 3822301 A1<br>claims, examples 1-23 | |
| JP 2020-105360 A | 09 July 2020 | (Family: none) | |
| JP 2019-189819 A | 31 October 2019 | CN 110408331 A<br>KR 10-2019-0125172 A<br>TW 202000825 A | |
| JP 2015-192109 A | 02 November 2015 | (Family: none) | |
| JP 2010-138259 A | 24 June 2010 | US 2011/0262746 A1<br>claims, examples 1-7<br>EP 2377904 A1<br>TW 201033318 A<br>CN 102245726 A | |
| JP 2009-292727 A | 17 December 2009 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2000044904 A